# EUROPEAN PATENT SPECIFICATION

(11) **EP 2 515 806 B1**
(45) Date of publication and mention of the grant of the patent: **18.10.2017**
(21) Application number: 10805786.0
(22) Date of filing: 21.12.2010
(51) Int. Cl.: A61K 9/00, A61K 9/127, A61F 6/14

(54) **IMPROVEMENTS TO FRAMELESS INTRAUTERINE DEVICES AND SYSTEMS**
VERBESSERUNGEN FÜR RAHMENLOSE INTRAUTERINE VORRICHTUNGEN UND SYSTEME
AMÉLIORATIONS APPORTÉES AUX DISPOSITIFS ET SYSTÈMES INTRA-UTÉRINS SANS CADRE

(30) Priority: 21.12.2009 WO PCT/EP2009/067709
(43) Date of publication of application: 31.10.2012
(73) Proprietor: PAT&Co bvba, 8810 Lichtervelde (BE)
(72) Inventor: WILDEMEERSCH, Dirk, B-Ghent (BE)
(74) Representative: Brantsandpatents bvba
(86) International application number: PCT/EP2010/070434
(87) International publication number: WO 2011/080164

(56) References cited:
- EP-A1- 2 308 428
- US-A- 4 708 134

## Description

The invention relates to a new and improved "frameless" copper-releasing or hormone-releasing intrauterine contraceptive device or system (IUD/IUS).
The known frameless copper intrauterine device, called GyneFix^{®}' has six copper tubes, each 5 mm long and 2.2 mm wide, threaded on a length of thread or suture material. This was generally disclosed in US 4,708,134 (which is regarded as the closest prior art) or EP 0191747. The proximal end of the device is provided with an anchoring means for its fixation to the uterine wall in order to prevent that the device is pushed out by the uterus.

Another known device or system is the frameless hormone-releasing FibroPlant^{®} which is similar to the GyneFix^{®} IUD but instead of copper cylinders, as the active component, is provided with a fiber or several fibers or loops held together in one assembly zone, and which releases the hormone in the uterine cavity. The hormone, such as steroid hormone, is a contraceptive and/or a therapeutic agent. An anchoring means similar to the above mentioned one is also provided for its fixation in the uterine wall. This was generally disclosed in US 5,433,218 or EP0445150.
These devices are remarkably well tolerated by any uterus, irrespective of its size and shape, due to the absence of a rigid frame, which eliminates dimensional incompatibility with the uterine cavity.

In order to retain the IUD/IUS in the uterine cavity, there is therefore a means to suspend the device to prevent its expulsion from the uterus. The preferred means is to suspend the IUD or IUS in the uterine cavity simply by providing a knot in the anchoring thread and then pushing such in the wall of the uterus by means of an applicator comprising, for example, a stylet-tip cooperating with a small loop extending from the knot.

A knot in the context of the present invention consists of a location of the thread were the thread has been deformed and/or multiply folded in such a way to produce a localised stable mass or lump of thread comprising at least a loop extending from this mass in order to provide a way for a hooking point of a stylet-tip during the insertion process. The mass of thread may be produced by intertwining and/or its stability enhanced by local fusion of the thread which is made at least partially of thermoplastic component. The dimension of the knot is larger than the diameter of the thread, for example 3 to 6 times larger, typically between 0.8 and 2.0 mm. The proximal end of the thread becomes a short appendix extending from the knot. The knot or deformation in the thread is small in volume and of a form suitable to allow its withdrawal under the effect of sufficient pulling, form the tissue of the uterus without damaging the latter.

Both active substances, copper and the hormone-releasing fibrous delivery system, can be visualized and their position in the uterine cavity can be checked by ultrasound.

The anchor in the tissue however cannot be visualized, or insufficiently, in many instances. The proper position of the anchor in the wall of the uterus is extremely important as the performance of the IUD/IUS depends on it. It can become expelled or the anchor can be inserted too deep, perforating the wall of the uterus. Perforation of the uterus is a serious adverse event which can lead to pain complaints, infection in the abdomen and pregnancy as there is no protection anymore if the IUD/IUS is not in situ.

In order to solve this problem and make it possible for the doctor to check the correct position of the anchor, according to the invention, a means has been provided to visualize the anchoring knot.

According to a preferred embodiment of the invention, there is indeed threaded a tiny metal tube on the anchoring thread immediately below the anchoring knot. Studies have shown that steel is more echogenic on ultrasound than copper and therefore the stainless steel tube can be kept extremely small. It is also important that the right material is used as the material should be biocompatible because the tiny steel tube will remain in the uterine wall for months or years until the IUD/IUS is removed.

It has been surprisingly found that this tiny metal tube in the fundus is well tolerated and does not affect the other properties of the frameless IUD/IUS.

Another aspect of the current improvement to frameless copper devices is related to the size of the copper elements loaded onto the IUD.

The abovementioned device known as GyneFix^{®} comprises copper cylinders and is very efficient as the inner surface of the copper cylinders is in contact with the uterine environment, thereby increasing the effective copper surface area, as there is no plastic frame. This, as mentioned, is different from other known copper IUDs.

This standard prior art "frameless" IUD has an effective copper surface area of approximately 330 mm2 and is a highly effective intrauterine contraceptive system. In randomized clinical trials, it has been shown to be more effective that the most effective copper IUD available on the market. In addition, with this IUD, because of its smaller size and flexibility, the effect on menstrual blood loss is reduced when compared with the larger, conventional copper-releasing IUDs.

An important drawback of IUDs is indeed their tendency to cause heavy, sometimes painful, menstrual bleeding. Heavy bleeding is the commonest cause for IUD discontinuation. Discontinuation rates for bleeding vary from 7.0 to 12.0 during the first year of use. In the USA, an estimate of 36% of IUD users terminate during the first year for reasons other than the desire for pregnancy, including heavy menstrual bleeding and pain. Heavy menstrual bleeding may have a deleterious effect on health among normally healthy women. This is especially the case in women living in deprived developing countries where increased menstrual blood loss may precipitate or aggravate iron deficiency anaemia. It has been reported that even minute increases in menstrual blood loss may adversely affect the health of women especially during long-term use of IUD contraception.
Anaemia is common according to a recent report of the WHO (World Health Organization). The incidence of anaemia in Western Europe and the USA is about 8%, in the other European countries about 20-30%, South America about 20-30%.

It has now been found that the use of a still reduced size of the contraceptive device produces surprisingly even less menstrual blood loss (MBL) than the already small frameless IUD previously known, with a copper surface area of about 330 mm².

According to another aspect of the invention it has therefore been found that reducing the size of a device of the prior art further results in an intrauterine device which is surprisingly still highly reliable and efficient, due to its high effective copper surface area, and is well tolerated, with minimal effect on menstrual blood loss, because of its very small size.

According to still another aspect of the invention, an IUD device is provided with a means to increase the release of copper from the smaller device.

It is known that noble metals such as gold and silver can influence the release of copper. This effect has already been described in the patent document PCT/HU90/00009. The essence of this patent is that the active body is made of at least two metals forming a plurality of galvanic cells in the uterine environment and is accomplished by making an alloy or microscopic mixture of the different metals.

It has been shown that it is especially desirable to design a device of which the active substance is copper and to add at least one metal which has a higher electropositivity such as gold. The copper elements then form the anode and the gold the cathode of the galvanic cell. The uterine environment functions thereby as the electrolyte.

With the GyneFix^{®}-type IUD of the present invention, the improvement is obtained not by providing an alloy or mixture but surprisingly it is simply accomplished by alternating copper cylinders with small gold cylinders, creating these galvanic cells. In this way, the device remains, as intended, highly flexible resulting in optimal tolerance by the patient.

Previous dissolution studies of the GyneFix^{®} 330 IUD yielded a minimal dissolution rate, per copper tube, of 2.4 mg per year. The addition of gold increases the copper ion release in the uterine environment thereby enhancing the contraceptive effectiveness based on the increased inactivation of the spermatozoa.

The primary aim of the present invention is to provide a means to make the current anchor visible on ultrasound and x-ray to allow the provider to check if the anchor is properly placed in the wall of the uterus since the performance of the frameless IUD/IUS depends on correct placement of the anchor. The visualization of the anchor thereby significantly increases the safety of the anchoring concept.

This goal is reached by adding a tubular metal element to the anchor fixed onto the anchoring thread just below the anchoring knot and/or on the appendix of the anchoring knot.

If a tube is threaded on the anchoring thread, this method of realization does not hinder the anchor for suspending the copper IUD or of the hormone-releasing IUS when inserted with an applicator into the musculature of the uterine fundus.

According to a particular embodiment, the anchoring knot may also be treated with metal dust attached or incorporated in the material of the anchoring knot or deformation of the thread serving as anchor. In this case the anchoring knot is preferably created by heat deformation during which small particles of metal are added.

The secondary aim of the present invention is to provide a copper IUD to minimize the impact on menstrual blood loss. This device has a smaller total (nominal) surface area but a large effective copper surface area, when compared to the effective copper surface area of the high-load conventional copper IUDs.

According to one characteristic of the invention, the total surface area of the IUD does not exceed 275 mm2. According to one preferred embodiment it will not exceed 200 mm2.

According to an additional characteristic of the invention, the effective copper surface area equals the nominal copper surface area which should be at least 200 mm2.

According to yet another aspect of the invention, the components are copper cylinders and are not longer than 6 to 8 mm in order to allow easier direct contact between the inner surface of the cylinders and the uterine environment.

According to yet another characteristic of the invention, all copper cylinders are kept in place by two small copper clips, one above and one below the copper cylinders, crimped onto the anchoring thread. The clips may be cylinders identical to the copper cylinders but which have been crimped. The design of the device and the manufacturing process may therefore be simplified.

According to still another additional characteristic of the invention, the total length of the closely approximated copper elements of the IUD is not longer than 2.6 cm.

According to still another additional characteristic of the invention, the total length of the IUD is not longer than 3.0 cm when the copper elements are freely separated.

According to an embodiment, the hollow elements are separated from each other by a small space to enhance the contact between the inner part of the cylinders and the uterine environment.

According to yet another characteristic of the invention, the hollow elements are not larger than 3.0 mm in diameter in order to allow easy insertion in the uterine cavity.

According to another characteristic of the invention, the inner diameter of the hollow elements is at least 1.6 mm in diameter to allow a high total (inner and outer) effective copper surface area similar to high-load copper IUDs.

The number of elements may be between 2 and 10, preferably between 3 to 6. Particularly preferred is a structure comprising 4 cylindrical elements, each approximately 5 mm in length, in a row of which the first and last have been crimped onto the anchoring thread.

According to another aspect of the invention there is provided a way to increase the copper release to enhance the inactivation by the copper ions on the sperm cells which is the main mechanism of action of copper IUDs. As mentioned before, copper and gold (or silver) enter into an electrochemical reaction. An alloy of copper and noble metal (silver, gold) creates a multitude of local tiny galvanic (alkaline) batteries when in contact with the uterine environment (which serves as an electrolyte). Copper act as an anode, while the noble metals (Ag, Au) serve as cathode. The resulting weak electrical activity has a negative effect on sperm cell motility, capacitation and survival, while the foreign body reaction, also stimulated electrically, increases leucocyte infiltration in the endometrium and further increases the number of macrophages in the uterine cavity (enhanced spermicidal effect). Silver and gold ions, dissolved in trace quantities, exhibit in situ bactericidal and fungicidal effects (oligodynamic effects), which are expected to lessen the risk of pelvic inflammatory disease (infection).

With the frameless IUD, the use of metal alloys is less practical than to use tubular elements which can simply be treaded on the anchoring suture. In view of the above considerations, practically, gold tubes with length varying between 1.6 and 2.0 mm are added between the copper tubes of the small IUD (GyneFix^{®} type) at three possible locations : first, below the first "crimped" copper tube; second, between the "loose" (noncrimped) copper tubes; and thirdlyabove the lower "crimped" copper tube.

According to a particular embodiment at least one short 2.0-3.0 mm gold tube is crimped onto the suture thread, for example one above and/or one below the four copper tubes.

An additional purpose of the invention may be to reduce the risk of expulsion in cases where the uterine wall is soft (e.g., postpartum) by the provision of biodegradable material added to the anchoring knot. This can be realized by putting a cap on the assembled anchoring knot. The cap covers preferably the upper part of the knot and leaves a loop extending therefrom for permitting the insertion process with an inserter. The biodegradable cap will degrade in weeks or a few months, leaving only the anchoring knot for retention of the contraceptive device in the uterine cavity at the time the uterus has involuted. Exemplary biodegrable material is a polylactide or polycaprolactone.

These and other characteristics of the invention will be more readily understood when referring to the description as well as the accompanying drawings which represent, merely by way of examples, several embodiments of the invention, and in which:
FIG.1 is an enlarged schematic sectional view of the anchor provided with a thin tube which is threaded just below the anchoring knot.
FIG. 2 represents the anchor as in Fig. 1 provided with a thin tube threaded and affixed onto the appendix of the anchor.
FIG. 3 is a schematic cross sectional view of the uterus with a preferred embodiment of an IUD according to the invention, and Fig. 4 is a schematic cross sectional view of the uterus with a preferred embodiment of a hormone-releasing IUS according to the invention.
FIG. 5 represents a view of a preferred embodiment of a copper-releasing IUD according to the invention (B), compared with a conventional copper-T IUD (A), showing the significantdifference in size between the two IUDs,
FIG. 6 is a transversal sectional view of a copper element of the invention,
FIG. 7 is another view of an embodiment according to the invention of which the copper elements are separated from each other to enhance the contact between the inner surface of the hollow cylinders and the uterine fluid,
FIG. 8 represents another view of embodiments according to the invention of which the copper elements are separated from each other by gold or silver tubes forming galvanic cells in succession,
FIG. 9 is a sectional schematic view of a mode of realization where a biodegradable cap is added and covers the anchoring knot for enhanced retention in the fundal part of the uterus.

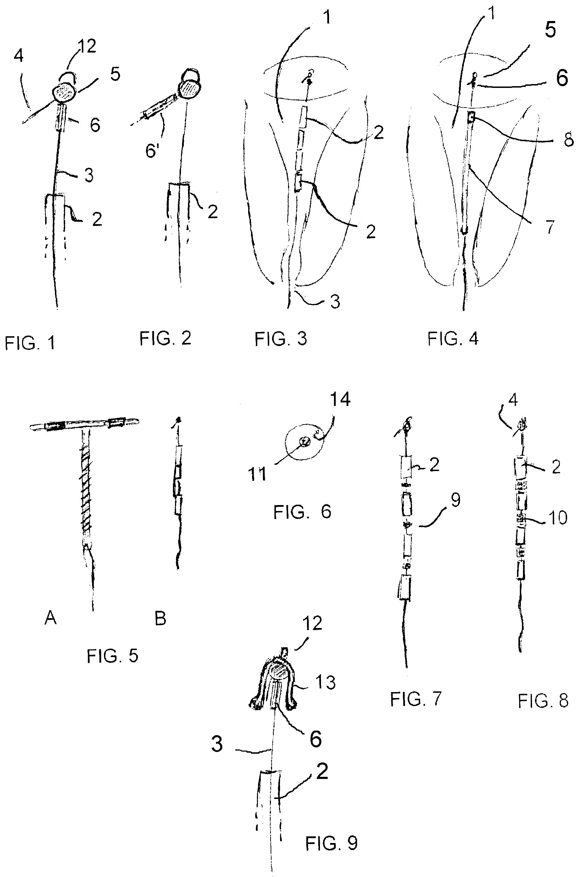

Referring now more particularly to Fig. 1, an anchor is provided by making a knot 5 in a suture thread 3 of which one of the ends is cut, leaving a relatively short appendix 4 extending from the knot. Below the knot, threaded onto the suture thread is a tiny tube 6 of maximum 2 mm long and less than double the size of the suture thread to allow its easy insertion in the wall of the uterus with a specially designed inserter. Also illustrated is a small loop 12 extending from the knot 5 and designed to cooperate with an inserter for pushing the not into the fundal tissue of the uterus.

According to another characteristic of the invention, the metal tube 6 is threaded on the appendix 4 of the anchoring knot 5 as shown in Fig. 2, and kept in place by slightly crimping the tube on the appendix.

Referring now particularly to FIG. 3, an IUD is made of a material which is physiologically active in the uterine cavity, consisting of 4 hollow longitudinal members 2, open at both ends, and arranged in a sequence in order to form a longitudinal flexible body. The elements 2 are threaded on a length of thread 3, for example a polypropylene suture material. The sleeve members or elements 2 are prevented from sliding off the material by the upper and lower sleeves which are at least partially crimped onto the thread. The proximal end of the thread is provided with a fixing means 5 which is pierced by about 1 cm in the fundal part of the uterine cavity 1 to secure the IUD therein and prevent expulsion. The resulting device has no rigid plastic body, making it a completely flexible and supple unit.

Preferably the IUD is made of hollow elements of which the total surface area is at least two times smaller when compared to a well-known conventional IUD (TCu380A) as shown in FIG. 5.

Fig. 4 shows the same anchoring technology as in Fig. 3, according to the invention, but the copper tubes are replaced by a fibrous delivery system 7 affixed to the anchoring thread by means of a metal cylinder 8 crimped onto the anchoring thread.

FIG. 6 is a transversal sectional view of an element of the invention, showing the possibility of exchange of contact between the inner surface 14 of the hollow cylinders and the uterine fluid. In the center is a cross sectional view of a metal element 11 consisting of a noble metal which forms a galvanic cell with a copper cylinder.

As shown in FIG. 7, which illustrates a IUD according to one embodiment, which comprises 4 hollow elements, open at both ends, separated from each other by a spacer 9, being provided crimped onto the thread or not, to enhance the contact with the uterine environment 11. According to one embodiment such crimped elements can be made of a noble metal.

Figure 8 is one of the preferred embodiments of the invention in order to increase the inactivating impact on sperm cells, bacteria and viruses. The cross sectional view shows a copper-releasing IUD according to the invention consisting of hollow copper elements, open at both ends, separated from each other by hollow gold elements creating relatively highly active galvanic cells due to the fact that all surfaces are open to the uterine environment, being the electrolyte.

Figure 9 is a sectional view of a preferred mode of realization of the "enhanced" anchor showing a cross sectional view of the anchoring knot with biodegradable bell-shaped cap 13 pushed over it.

The present invention discloses, among others, in a non limitative way :
- an intrauterine contraceptive device (IUD) comprising: a plurality of elongated hollow, metabolically active members, open at both ends and arranged longitudinally onto a thread, one end of which comprises an anchoring means to retain to the fundal part of the uterine cavity , such device forming a non-rigid structure of limited dimensions, characterised in that: the structure has a total surface area not exceeding 275 mm²; the length of the structure, when the hollow members are closely approximated is less than 2.6 cm, and not more than 3.0 cm when the members are freely separated along said thread, and its diameter is not more than 3.30 mm, preferably not more than 3.00 mm; and the hollow members have an inner diameter of at least 1.6 mm but less than 2.5 mm, and/or
- an intrauterine contraceptive device for insertion in the uterus comprising: a plurality of hollow, metabolically active elements, arranged longitudinally to form a non-rigid structure of limited dimensions; a structure of which the elements are copper cylinders are at least 2.4 mm in diameter and maximum 2.8 mm in length; a structure of which the wall thickness of each or part of the copper cylinders is at least 0.4 mm, with the following additional optional features :
- the hollow members are hollow cylinders or sleeves, the length thereof being equal to or less than 8 mm;
- the wall of the hollow members is partially cut out or perforated;
- the hollow members are made of copper;
- one or some of the hollow members (2) are made of copper and the other(s) of gold and/or silver;
- the hollow members 2 are kept in place by two small crimped clips, one above and one below the hollow elements;
- the clips are also hollow elements, possibly of shorther length, having been crimped or flattened onto the thread;
- the number of members 2 is between 3 to 8, preferably around 4;
- the total copper load is minimally 350 mg;
- retaining thread is made of polypropylene and/or has at least 0-gauge size US;
- the total length of the hollow members does not exceed 2.5 cm;
- the effective copper surface area is between 220 mm² and 280 mm²;
- there is provided a hollow member or thin tube which is threaded just below the anchoring knot;
- the hollow member is made of copper and has a length between 1 and 9 mm and a diameter which is between 0.1 and 0.3 mm larger than the size of the suture thread (3) on which the tiny metal tube is threaded;
- there is provided a further crimped hollow member at an appendix of the anchoring means,
- the hollow members are copper cylinders and the wall thickness of each or part of the copper cylinders is at least 0.4 mm.

The invention has been described and illustrated merely by way of examples which are in no way restrictive. Novel features can be taken separately or in any possible combination. More particularly the present invention discloses generally, separately or in any combination a frameless intrauterine device having
- a metallic element such as a steel cylinder at the anchoring location insertable inside the tissue of the uterus;
- very small dimensions compared to the prior art device;
- noble metal cylinders, such as gold or silver cylinders, threaded adjacent to copper elements;
- a knot for anchoring the device which is capped by a biodegradable component, such knot being not necessarily completly embedded in the biodegrable component.
Numerous changes in the detailed conception of the invention may be made without departing from the scope of the invention as defined in the annexed claim 1.

## Claims

1. A frameless copper-releasing contraceptive IUD or a hormone-releasing contraceptive IUS, comprising a retaining thread (3), such as suture thread, supporting the active component(s) (2, 6) and an anchor insertable into the wall of an uterus (1) with a specially designed applicator or inserter in order to secure the IUD/IUS in the uterine cavity, said anchor consisting of a knot (5) made at one end of the thread (3) **characterized by** a tiny metal tube (6) threaded onto the thread and fixed immediately below or above the anchoring knot (5) to make the insertable anchor visible in the uterine wall on ultrasound and/or x-ray.

2. An IUD according to claim 1 wherein the tube (6) is made of stainless steel.

3. An IUD according to claim 1 or 2 wherein the tube (6) is fixed by crimpage onto the thread (3).

4. An IUD according to any of the previous claims **characterized in that** the anchoring knot is provided with a tightly fitting ogival, bell or cone-shaped cap (13) consisting of a biodegradable polymer.

5. An IUD according to any of the claims 1 to 4 wherein the anchoring thread (3) carries an active element which consists of a succession of threaded copper cylinders (2).

6. An IUD according to any of the previous claims **characterized in that** the tube (6) which is threaded just below the anchoring knot consists of copper or biocompatible stainless steel and has a length which is not longer than 2 mm and a diameter which is less than twice the diameter of the suture thread to allow easy insertion in the wall of the uterus(1).

7. An IUD according to any of the previous claims, **characterized in that** there is provided a crimped hollow member (6') at the appendix of the anchoring means (5).

8. An intrauterine contraceptive device (IUD) according to any of the claims 1 to 7 comprising a plurality of hollow, metabolically active elements (2), open at both ends and arranged longitudinally onto a thread (3), such device forming a non-rigid structure of limited dimensions, **characterised in that** the structure has a total surface area not exceeding 275 mm²; the length of the structure, when the elements are closely approximated is less than 2.6 cm, and not more than 3.0 cm when the elements (2) are freely separated along said thread (3), and its diameter is not more than 3.30 mm, preferably not more than 3.00 mm; and the hollow elements have an inner diameter of at least 1.4 mm but less than 2.5 mm.

9. An IUD according to claim 8 wherein the hollow elements (2) are hollow cylinders or sleeves, the length thereof being equal or less than 8 mm.

10. An IUD according to any of the previous, claims **characterized in that** one or some of the hollow members (2) are made of copper and the other(s) (10) of gold and/or silver and are positioned adjacent to a copper element (2).

11. An IUD according to any of the previous claims wherein the number of elements (2) is between 3 to 8, preferably around 4.

12. An IUD according to to any of the previous claims, **characterized in that** the hollow members (2) are affixed to a retaining thread which is made of polypropylene and which has at least 00-gauge size.

13. An IUD according to to any of the previous claims, **characterized in that** the total length of the hollow members (2) does not exceed 2.5 cm.

14. An IUD according to any of the previous claims , **characterized in that** the effective copper surface area is between 200 mm² and 280 mm².

15. An IUD according to claim 1 to 4 wherein the anchoring thread (3) carries an active element (7) which consists of a hormone-releasing fiber able to release a hormone in the uterine cavity for contraception and gynecological treatment and which is fixed to the anchoring thread (3).

## Patentansprüche

1. Ein rahmenloses Kupfer-freisetzendes kontrazeptives Intrauterinpessar (IUP) oder eines Hormon-freisetzendes kontrazeptives Intrauterines System (IUS), umfassend einen Haltefaden (3), wie Nahtfaden, der die aktive(n) Komponente(n) (2, 6) unterstützt und einen Anker einführbar in die Wand eines Uterus (1) mit einem speziell entwickelten Applikator oder Inserter, um das IUP/IUS in der Gebärmutterhöhle zu sichern, der Anker bestehend aus einem an einem Ende des Fadens gemachten (3) Knoten (5), **gekennzeichnet durch** ein kleines Metallrohr (6), auf den Faden eingefädelt und unmittelbar unterhalb oder oberhalb des Verankerungsknotens (5) fixiert, um den einführbaren Anker in der Uteruswand auf dem Ultraschall und/oder Röntgenstrahl sichtbar zu machen.

2. Eines IUP nach Anspruch 1, wobei das Rohr (6) aus Edelstahl hergestellt ist.

3. Eines IUP nach Anspruch 1 oder 2, wobei das Rohr (6) durch Crimpen auf den Faden (3) fixiert ist.

4. Eines IUP nach einem der vorhergehenden Ansprüche, **dadurch gekennzeichnet, dass** der Verankerungsknoten mit einer fest sitzenden spitzbogenförmigen, glockenförmigen oder kegelförmigen Kappe (13) bestehend aus einem biologisch abbaubaren Polymer versehen ist.

5. Eines IUP nach einem der Ansprüche 1 bis 4, wobei der Verankerungsfaden (3) ein aktives Element trägt, das aus einer Folge von Kupferzylindern mit Gewinde (2) besteht.

6. Eines IUP nach einem der vorhergehenden Ansprüche, **dadurch gekennzeichnet, dass** das Rohr (6), das unmittelbar unterhalb des Verankerungsknotens eingefädelt ist, aus Kupfer oder biokompatibler Edelstahl besteht und eine Länge, die nicht mehr als 2 mm ist und einen Durchmesser aufweist, der weniger ist als das Doppelte des Durchmessers des Nahtfadens, um eine einfache Einführung in die Wand des Uterus (1) zu ermöglichen.

7. Eines IUP nach einem der vorhergehenden Ansprüche, **dadurch gekennzeichnet, dass** an dem Anhang des Verankerungsmittels (5) ein gequetschtes hohles Teil (6') vorgesehen ist.

8. Eine intrauterine kontrazeptive Vorrichtung (IUP) nach einem der Ansprüche 1 bis 7, umfassend eine Vielzahl von hohlen, metabolisch aktiven Elementen (2), an beiden Enden offen und in Längsrichtung auf einen Faden (3) angeordnet, eine derartige Vorrichtung bildet eine nicht-starre Struktur mit begrenzten Abmessungen, **dadurch gekennzeichnet, dass** die Struktur eine Gesamtfläche von nicht mehr als 275 mm² hat; die Länge der Struktur, wenn die Elemente eng angenähert sind, ist weniger als 2,6 cm, und nicht mehr als 3,0 cm wenn die Elemente (2) frei getrennt entlang dem Faden (3) sind, und dessen Durchmesser nicht mehr als 3,30 mm ist, vorzugsweise nicht mehr als 3,00 mm; und die Hohlelemente einen Innendurchmesser von mindestens 1,4 mm, aber weniger als 2,5 mm, aufweisen.

9. Eines IUP nach Anspruch 8, wobei die Hohlelemente (2) Hohlzylinder oder Hülsen sind, deren Länge gleich oder weniger als 8 mm ist.

10. Eines IUP nach einem der vorhergehenden Ansprüche, **dadurch gekennzeichnet, dass** eines oder ein Teil der Hohlkörper (2) aus Kupfer und das/die andere (10) aus Gold und/oder Silber hergestellt sind und angrenzend an ein Kupferelement (2) positioniert sind.

11. Eines IUP nach einem der vorhergehenden Ansprüche, wobei die Anzahl der Elemente (2) zwischen 3 bis 8, vorzugsweise etwa 4, ist.

12. Eines IUP nach einem der vorhergehenden Ansprüche, **dadurch gekennzeichnet, dass** die Hohlkörper (2) an einem Haltefaden, der aus Polypropylen hergestellt ist und mindestens eine Gauge-Größe von 00 hat, befestigt sind.

13. Eines IUP nach einem der vorhergehenden Ansprüche, **dadurch gekennzeichnet, dass** die Gesamtlänge der Hohlkörper (2) 2,5 cm nicht übersteigt.

14. Eines IUP nach einem der vorhergehenden Ansprüche, **dadurch gekennzeichnet, dass** die effektive Kupferoberfläche zwischen 200 mm² und 280 mm² liegt.

15. Eines IUP nach Anspruch 1 bis 4, wobei das Verankerungsfaden (3) ein aktives Element (7) trägt, das aus einer Hormon-freisetzenden Faser besteht, die fähig ist, ein Hormon ein Hormon in der Gebärmutterhöhle freizusetzen für die Kontrazeption und gynäkologische Behandlung, und das an dem Verankerungsfaden (3) fixiert ist.

## Revendications

1. Un DIU contraceptif libérant du cuivre sans cadre ou un SIU contraceptif libérant des hormones, comprenant un fil de retenue (3), tel qu'un fil de suture, supportant le(s) composant (s) actif(s) (2, 6) et une ancre insérable dans la paroi d'un utérus (1) avec un applicateur ou un inséreur spécialement conçu afin de sécuriser le DIU/SIU dans la cavité utérine, ladite ancre constituée d'un noeud (5) fait à une extrémité du fil (3) **caractérisé par** un minuscule tube métallique (6), fileté sur le fil et fixé immédiatement au-dessous ou au-dessus du noeud d'ancrage (5) pour rendre l'ancre insérable visible dans la paroi utérine sur des ultrasons et/ou aux rayons X.

2. Un DIU selon la revendication 1, dans lequel le tube (6) est fait d'acier inoxydable.

3. Un DIU selon la revendication 1 ou 2, dans lequel le tube (6) est fixé par sertissage sur le fil (3).

4. Un DIU selon l'une quelconque des revendications précédentes, **caractérisé en ce que** le noeud d'ancrage est pourvu d'un capuchon (13) en forme ogivale, de cloche ou de cône étroitement ajusté constitué d'un polymère biodégradable.

5. Un DIU selon l'une quelconque des revendications 1 à 4, dans lequel le fil d'ancrage (3) porte un élément actif qui consiste en une succession de cylindres en cuivre filetés (2).

6. Un DIU selon l'une quelconque des revendications précédentes, **caractérisé en ce que** le tube (6) qui est fileté juste au-dessous du noeud d'ancrage se compose de cuivre ou d'acier inoxydable biocompatible et a une longueur n'excédant pas 2 mm et un diamètre qui est moins de deux fois le diamètre du fil de suture pour permettre une insertion facile dans la paroi de l'utérus (1).

7. Un DIU selon l'une quelconque des revendications précédentes, **caractérisé en ce qu'**il est prévu un membre creux serti (6') à l'appendice du moyen d'ancrage (5).

8. Un dispositif contraceptif intra-utérin (DIU) selon l'une quelconque des revendications 1 à 7, comprenant une pluralité d'éléments creux métaboliquement actifs (2), ouverts aux deux extrémités et disposés longitudinalement sur un fil (3), ce dispositif formant une structure non rigide de dimensions limitées, **caractérisé en ce que** la structure a une surface totale n'excédant pas 275 mm²; la longueur de la structure, lorsque les éléments sont étroitement approchés, est inférieure à 2,6 cm, et pas plus de 3,0 cm lorsque les éléments (2) sont séparés librement le long dudit fil (3), et son diamètre n'est pas supérieur à 3,30 mm, de préférence pas supérieur à 3,00 mm; et les éléments creux ont un diamètre intérieur d'au moins 1,4 mm mais inférieur à 2,5 mm.

9. Un DIU selon la revendication 8, dans lequel les éléments creux (2) sont des cylindres ou des manchons creux, dont la longueur est égale ou inférieure à 8 mm.

10. Un DIU selon l'une quelconque des revendications précédentes, **caractérisé en ce que** l'un ou quelques des éléments creux (2) sont en cuivre et les autre(s) (10) en or et/ou en argent et sont positionnés adjacents à un élément en cuivre (2).

11. Un DIU selon l'une quelconque des revendications précédentes, dans lequel le nombre d'éléments (2) est compris entre 3 et 8, de préférence environ 4.

12. Un DIU selon l'une quelconque des revendications précédentes, **caractérisé en ce que** les éléments creux (2) sont fixés à un fil de retenue qui est en polypropylène et qui a au moins une taille de jauge 00.

13. Un DIU selon l'une quelconque des revendications précédentes, **caractérisé en ce que** la longueur totale des éléments creux (2) n'excède pas 2,5 cm.

14. Un DIU selon l'une quelconque des revendications précédentes, **caractérisé en ce que** la surface de cuivre effective est comprise entre 200 mm² et 280 mm².

15. Un DIU selon la revendication 1 à 4, dans lequel le fil d'ancrage (3) porte un élément actif (7) qui consiste en une fibre libérant des hormones capable de libérer une hormone dans la cavité utérine pour la contraception et le traitement gynécologique et qui est fixée au fil d'ancrage (3).
